(19) Europäisches Patentamt
European Patent Office
Office européen des brevets

(11) EP 0 770 050 B1

(12) # FASCICULE DE BREVET EUROPEEN

(45) Date de publication et mention de la délivrance du brevet:
**16.08.2001 Bulletin 2001/33**

(21) Numéro de dépôt: **96916192.6**

(22) Date de dépôt: **10.05.1996**

(51) Int Cl.⁷: **C07C 45/46**, C07C 49/84, B01J 29/08

(86) Numéro de dépôt international:
**PCT/FR96/00716**

(87) Numéro de publication internationale:
**WO 96/35655 (14.11.1996 Gazette 1996/50)**

(54) **PROCEDE D'ACYLATION D'ETHERS AROMATIQUES**

VERFAHREN ZUR ACYCLIERUNG VON AROMATISCHEN ETHERN

AROMATIC ETHER ACYLATION PROCESS

(84) Etats contractants désignés:
**BE CH DE ES FR GB IT LI NL**

(30) Priorité: **12.05.1995 FR 9505682**

(43) Date de publication de la demande:
**02.05.1997 Bulletin 1997/18**

(73) Titulaires:
- **RHODIA CHIMIE**
  **92408 Courbevoie Cédex (FR)**
- **INSTITUT FRANCAIS DU PETROLE**
  **92506 Rueil Malmaison Cédex (FR)**

(72) Inventeurs:
- **SPAGNOL, Michel**
  **F-69003 Lyon (FR)**
- **GILBERT, Laurent**
  **F-75015 Paris (FR)**
- **BENAZZI, Eric**
  **F-78360 Montesson (FR)**
- **MARCILLY, Christian**
  **F-78800 Houilles (FR)**

(74) Mandataire: **Dutruc-Rosset, Marie-Claude et al**
**Rhodia Services,**
**Direction de la Propriété Industrielle,**
**40, rue de la Haie-Coq**
**93306 Aubervilliers Cedex (FR)**

(56) Documents cités:

| | |
|---|---|
| **EP-A- 0 334 096** | **EP-A- 0 407 203** |
| **EP-A- 0 455 332** | **EP-A- 0 459 495** |
| **FR-A- 2 667 063** | **US-A- 3 130 007** |

- **APPL. CATAL. (APCADI,01669834);89; VOL.49 (1); PP.109-23, CSIC;INST. CATAL. PETROLEOQUIM.; MADRID; 28006; SPAIN (ES), XP002012280 CORMA A ET AL: "Design of synthetic zeolites as catalysts in organic reactions: acylation of anisole by acyl chlorides or carboxylic acids over acid zeolites"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 770 050 B1

## Description

La présente invention a pour objet un procédé d'acylation d'un éther aromatique.

L'invention vise préférentiellement un procédé d'acylation d'un éther aromatique substitué et plus particulièrement du vératrole.

L'invention s'applique à la préparation d'alkylcétones alkoxyaromatiques.

Les procédés classiques d'acylation des composés aromatiques, notamment des éthers de phénols consistent à effectuer une réaction d'acylation de type Friedel-Crafts.

On fait réagir le composé aromatique et un agent d'acylation, en présence d'un catalyseur qui est généralement le chlorure d'aluminium.

Une illustration de ce type de procédé est donnée par les travaux de C. KURODA et coll. [Sci. Papers Inst. Phys. Chem. Res. 18, pp. 51-60 (1932)] qui ont décrit la préparation de méthoxyacétophénones, par réaction d'un composé aromatique porteur de 1 à 3 groupes méthoxy, avec du chlorure d'acétyle, en présence de chlorure d'aluminium.

Toutefois, la mise en oeuvre du chlorure d'aluminium présente de nombreux inconvénients. Le chlorure d'aluminium est un produit corrosif et irritant. De plus, il est nécessaire de mettre en oeuvre une quantité importante de chlorure d'aluminium au moins égale à la stoechiométrie, par suite de la complexation de la cétone formée. En conséquence, le chlorure d'aluminium n'est donc pas un vrai catalyseur.

En fin de réaction, il est nécessaire d'éliminer le chlorure d'aluminium du milieu réactionnel en faisant une hydrolyse acide ou basique.

Cette technique d'hydrolyse implique l'addition d'eau dans le milieu réactionnel ce qui complique notablement la mise en oeuvre du procédé car le cation métallique, et plus particulièrement le cation aluminium forme alors en présence d'eau, des complexes polyoxo- et/ou polyhydroxo- d'aluminium de consistance laiteuse, difficiles ensuite à séparer. Il en résulte la nécessité de faire un traitement long et coûteux comportant après l'hydrolyse, une extraction de la phase organique, une séparation des phases aqueuse et organique, voire-même un séchage de cette dernière. La séparation du chlorure d'aluminium est donc longue et coûteuse.

Par ailleurs, se pose le problème des effluents aqueux salins qu'il faut ensuite neutraliser ce qui impose une opération supplémentaire.

De plus, le chlorure d'aluminium ne peut être recyclé du fait de son hydrolyse.

Pour pallier cet inconvénient, on a proposé de faire la réaction en présence de catalyseurs hétérogènes.

Ainsi, depuis une dizaine d'années, on a préconisé de mettre en oeuvre, les zéolithes, comme catalyseurs d'acylation.

Prins et coll. ont décrit l'acétylation de l'anisole, par l'anhydride acétique [9$^{th}$ International Zeolite Congress - Montréal Congrès (1992)], en présence de zéolithes telles que zéolithe β ou zéolithe USY. Il est à noter que les zéolithes β donnent des résultats plus intéressants tant en ce qui concerne le taux de conversion que le rendement réactionnel.

On a divulgué selon EP-A-0279322, la réaction en phase vapeur, d'un composé aromatique (vératrole) avec un dérivé d'acide carboxylique, en présence d'une zéolithe sous forme H telle que mordénite, faujasite et ZSM-5.

On a décrit dans EP-A-0334096, un procédé d'acylation d'un éther aromatique (anisole). Une multitude de zéolithes sont citées mais il n'est pas fait mention de caractéristiques de zéolithes.

Par ailleurs, on a décrit dans EP-A-0407203, un procédé de désalumination d'une zéolithe qui consiste à la soumettre à un traitement hydrothermal.

Il s'avère que la mise en oeuvre des zéolithes comme catalyseurs de réaction faisant intervenir des molécules encombrées pose un problème à l'Homme du Métier en ce sens que la sélectivité et le rendement de la réaction ne sont pas satisfaisants.

L'objet de la présente invention est de fournir un procédé permettant d'obvier aux inconvénients précités.

Il a maintenant été trouvé et c'est ce qui constitue l'objet de la présente invention, un procédé d'acylation d'un éther aromatique qui consiste à faire réagir ledit éther avec un agent d'acylation, en présence d'un catalyseur zéolithique, ledit procédé étant caractérisé par le fait que l'on conduit la réaction d'acylation en présence d'une quantité efficace d'un catalyseur comprenant une zéolithe de type faujasite ou zéolithe Y ayant les caractéristiques physicochimiques suivantes :

- un rapport atomique dénommé "Si/$Me^1$ global", entre le nombre d'atomes de l'élément silicium et le nombre d'atomes de tout élément trivalent $Me^1$ contenu dans la zéolithe, compris entre 2,4 et 90, de préférence entre 2,4 et 75 et de manière encore plus préférée, entre 2,4 et 60,
- une teneur en métal alcalin $Me^2$ telle que le rapport atomique $Me^2/Me^{1(IV)}$, entre le nombre d'atomes de métal alcalin $Me^2$ et le nombre d'atomes de tout élément trivalent $Me^{1(IV)}$ inclus dans le réseau zéolithique, est inférieur à 0,2, de préférence inférieur à 0,1 et de manière encore plus préférée, inférieur à 0,05.

Par "$Me^1$", on désigne tout élément ayant un degré d'oxydation de +3 et notamment, l'aluminium, la gallium, le fer, le bore et leurs mélanges, et de préférence, l'aluminium.

Par "$Me^2$", on entend un métal choisi dans le groupe des éléments de la colonne 1a et leurs mélanges, de préférence les métaux alcalins tels que le lithium, le sodium, le potassium, le rubidium et le césium. $Me^2$ représente de préférence, le sodium ou le potassium.

Par "$Me^{1(IV)}$", on désigne tout élément ayant un degré d'oxydation de +3 et notamment, l'aluminium, la gallium, le fer ou le bore et leurs mélanges, et de préférence, l'aluminium, présent dans le réseau zéolithique.

Pour la définition des éléments, on se réfère ci-après à la Classification périodique des éléments publiée dans le Bulletin de la Société Chimique de France, n°1 (1966).

La zéolithe de type faujasite ou Y mise en oeuvre préférentiellement présente un rapport atomique dénommé "$Si/Me^{1(IV)}$ de charpente zéolithique", entre le nombre d'atomes de l'élément silicium et le nombre d'atomes de tout élément trivalent $Me^{1(IV)}$ inclus dans le réseau zéolithique compris entre 5 et 100, de préférence entre 6 et 80 et de manière encore plus préférée, entre 8 et 60.

Les paramètres physico-chimiques caractérisant la zéolithe mise en oeuvre dans le procédé de l'invention sont déterminés selon les méthodes données ci-après.

Le rapport atomique "$Si/Me^1$ global" de la zéolithe est déterminé notamment par fluorescence X.

Le rapport atomique "$Si/Me^{1(IV)}$" de la charpente de la zéolithe est déterminé par l'une des méthodes connues de l'homme du métier, à savoir et à titre d'exemples, la diffraction des rayons X qui, par mesure du paramètre de maille de la zéolithe, permet, en utilisant la relation de Fichtner-Schmittler (Fichtner-Schmittler, H., Loshe, U., Engelhardt, G. et Patzelova, V. Cryst. Rest. Tech. 1984, 1984, 19, K1) d'accéder au rapport "$Si/Me^{1(IV)}$" de charpente, la RMN du solide du $^{29}Si$ et la spectroscopie infrarouge.

Le catalyseur mis en oeuvre dans le procédé de l'invention comprend une phase active qui est une zéolithe de type faujasite ou Y ayant certaines caractéristiques bien définies. Ainsi, elle présente une faible teneur en élément $Me^1$, qui est de préférence, l'aluminium.

A l'état déshydraté, la zéolithe Y ou zéolithe de type faujasite a, plus précisément, une composition chimique correspondant à la formule empirique suivante :

$$(1\text{-}x)\ H_2O,\ x[(Me^2)_2O],\ [(Me^1)_2O_3],\ 2n\ SiO_2 \qquad (F1)$$

dans ladite formule:

- $Me^1$ et $Me^2$ ayant la signification donnée ci-dessus,
- n, égal au rapport atomique "$Si/Me^1$ global" défini précédemment, est compris entre 2,4 et 90, de préférence entre 2,4 et 75 et de manière encore plus préférée, entre 2,4 et 60,
- x, égal au rapport atomique $Me^2/Me^{1(IV)}$ défini précédemment, est inférieur à 0,2, de préférence inférieur à 0,1 et de manière encore plus préférée, compris entre 0 et 0,05.

Le paramètre de maille *a* de la zéolithe mise en oeuvre dans le procédé de l'invention est inférieur à 24,50 Angström, de préférence, compris entre 24,23 et 24,42 Angström.

Les zéolithes Y, de type faujasite sont décrites dans la littérature [cf. Atlas of zeolites structure types by W. M. Meier and D. H. Oison published by the Structure Commission of the International Zeolite Association (1978) ou brevet US 3 130 007].

Afin de mettre en oeuvre une zéolithe Y répondant aux caractéristiques précitées, il peut être nécessaire d'effectuer un traitement de désalumination de la zéolithe de telle sorte que les rapports atomiques $Si/Me^1$ définis précédemment soient compris dans les intervalles précités.

Ainsi, on peut mettre en oeuvre les méthodes connues de l'homme du métier parmi lesquelles on peut citer, à titre d'exemples, et de manière non exhaustive, les calcinations en présence de vapeur, les calcinations en présence de vapeur d'eau suivies d'attaques par des acides minéraux ($HNO_3$, HCl...), les traitements directes de désalumination par des réactifs tels que le tétrachlorure de silicium ($SiCl_4$), l'hexafluorosilicate d'ammonium ($(NH_4)_2SiF_6$), l'éthylènediaminetétracétique (EDTA) ainsi que sa forme mono- ou disodique. On peut également faire un traitement de désalumination par attaque acide directe par des solutions d'acides minéraux tels que par exemple, l'acide chlorhydrique, l'acide nitrique, l'acide sulfurique ou d'acides organiques comme notamment l'acide acétique, l'acide oxalique.
Par ailleurs, toute combinaison des méthodes de désalumination précitées est aussi possible.

La zéolithe constitue la phase catalytique. Elle peut être utilisée seule ou en mélange avec une matrice minérale. Dans la description, on désignera par "catalyseur", le catalyseur réalisé entièrement en zéolithe ou en mélange avec une matrice préparée selon des techniques connues de l'Homme du métier.

A cet effet, la matrice peut être choisie parmi les oxydes de métaux, tels que les oxydes d'aluminium, de silicium et/ou de zirconium, ou encore parmi les argiles et plus particulièrement, le kaolin, le talc ou la montmorillonite.
Dans le catalyseur, la teneur en phase active représente de 5 à 100 % du poids du catalyseur.
Les catalyseurs peuvent se présenter sous différentes formes dans le procédé de l'invention : poudre, produits mis en forme tels que granulés (par exemple, extrudés ou billes), pastilles, qui sont obtenus par extrusion, moulage, compactage ou tout autre type de procédé connu. En pratique, sur le plan industriel, ce sont les formes de granulés ou de billes qui présentent le plus d'avantages tant sur le plan de l'efficacité que sur le plan de commodité de mise en oeuvre.
Comme mentionné précédemment, le procédé de l'invention convient pour effectuer une réaction d'acylation sur un éther aromatique, de préférence, substitué.
Dans l'exposé qui suit de la présente invention, on désigne par "éther aromatique", un composé aromatique dont un atome d'hydrogène directement lié au noyau aromatique est remplacé par un groupe éther et par "aromatique", la notion classique d'aromaticité telle que définie dans la littérature, notamment par Jerry MARCH, Advanced Organic Chemistry, 4$^{ème}$ édition, John Wiley and Sons, 1992, pp. 40 et suivantes.
On entend par "éther aromatique substitué", un éther aromatique comprenant au moins un autre substituant sur le noyau aromatique, de préférence en position ortho.
Plus précisément, la présente invention a pour objet un procédé d'acylation d'un éther aromatique de formule générale (I) :

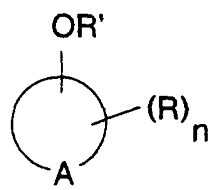

(I)

dans laquelle :

- A symbolise le reste d'un cycle formant tout ou partie d'un système carbocyclique aromatique, monocyclique ou polycyclique, système comprenant au moins un groupe OR' : ledit reste cyclique pouvant porter un ou plusieurs substituants,
- R représente un ou plusieurs substituants, identiques ou différents,
- R' représente un radical hydrocarboné ayant de 1 à 24 atomes de carbone, qui peut être un radical aliphatique acyclique saturé ou insaturé, linéaire ou ramifié ; un radical cycloaliphatique saturé, insaturé ou aromatique, monocyclique ou polycyclique ; un radical aliphatique saturé ou insaturé, linéaire ou ramifié, porteur d'un substituant cyclique,
- R' et R peuvent former un cycle comprenant éventuellement un autre hétéroatome,
- n est un nombre inférieur ou égal à 4.

Dans le présent texte, on désigne, de manière simplifiée, par "groupes alkoxy", les groupes du type R'-O- dans lesquels R' a la signification donnée précédemment. R' représente donc aussi bien un radical aliphatique acyclique ou cycloaliphatique, saturé, insaturé ou aromatique qu'un radical aliphatique saturé ou insaturé porteur d'un substituant cyclique.
L'éther aromatique qui intervient dans le procédé de l'invention répond à la formule (I) dans laquelle R' représente un radical aliphatique acyclique, saturé ou insaturé, linéaire ou ramifié.
Plus préférentiellement, R' représente un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome (par exemple, l'oxygène), par un groupe fonctionnel (par exemple -CO-) et/ou porteuse d'un substituant (par exemple, un halogène).
Le radical aliphatique acyclique, saturé ou insaturé, linéaire ou ramifié peut être éventuellement porteur d'un substituant cyclique. Par cycle, on entend de préférence, un cycle carbocyclique saturé, insaturé ou aromatique, de préférence cycloaliphatique ou aromatique notamment cycloaliphatique comprenant 6 atomes de carbone dans le cycle ou benzénique.
Le radical aliphatique acyclique peut être relié au cycle par un lien valentiel, un hétéroatome ou un groupe fonctionnel et des exemples sont donnés ci-dessus.
Le cycle peut être éventuellement substitué et à titre d'exemples de substituants cycliques, on peut envisager,

entre autres, les substituants tels que R dont la signification est précisée pour la formule (Ia).

R' peut représenter également un radical carbocyclique saturé ou comprenant 1 ou 2 insaturations dans le cycle, ayant généralement de 3 à 8 atomes de carbone, de préférence, 6 atomes de carbone dans le cycle ; ledit cycle pouvant être substitué avec des substituants tels que R.

R' peut représenter également un radical carbocyclique aromatique, de préférence monocyclique ayant généralement au moins 4 atomes de carbone, de préférence, 6 atomes de carbone dans le cycle ; ledit cycle pouvant être substitué avec des substituants tels que R.

Le procédé de l'invention s'applique tout particulièrement aux éthers aromatiques de formule (I) dans laquelle R' représente un radical alkyle linéaire ou ramifié ayant de 1 à 4 atomes de carbone ou un radical phényle.

Comme exemples de radicaux R' préférés selon l'invention, on peut citer les radicaux méthyle et éthyle.

Dans la formule générale (I) des éthers aromatiques, le reste A peut représenter le reste d'un composé carbocyclique aromatique, monocyclique ayant au moins 4 atomes de carbone et de préférence 6 atomes de carbone ou le reste d'un composé carbocyclique polycyclique qui peut être constitué par au moins 2 carbocycles aromatiques et formant entre eux des systèmes ortho- ou ortho- et péricondensés ou par au moins 2 carbocycles dont au moins l'un d'entre eux est aromatique et formant entre eux des systèmes ortho- ou ortho- et péricondensés. On peut citer plus particulièrement, un reste naphtalénique.

Le reste A peut porter un ou plusieurs substituants sur le noyau aromatique.

Des exemples de substituants R sont donnés ci-après mais cette liste ne présente pas de caractère limitatif. N'importe quel substituant peut être présent sur le cycle dans la mesure où il n'interfère pas au niveau du produit désiré.

Le reste A pouvant entre autres porter plusieurs groupes alkoxy, il est possible selon le procédé de l'invention d'acyler des composés polyalkoxylés.

Le procédé de l'invention s'applique plus particulièrement, aux éthers aromatiques de formule (Ia) :

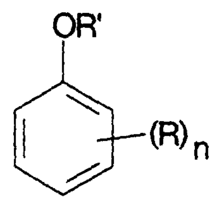

(Ia)

dans laquelle :

- n est un nombre inférieur ou égal à 4, de préférence égal à 1 ou 2,
- le radical R' représente un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle ou un radical phényle,
- le ou les radicaux R représentent l'un des atomes ou groupes suivants :
    - . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
    - . un radical alcényle linéaire ou ramifié ayant de 2 à 6 atomes de carbone, de préférence, de 2 à 4 atomes de carbone, tel que vinyle, allyle,
    - . un radical cyclohexyle ou benzyle,
    - . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
    - . un groupe acyle ayant de 2 à 6 atomes de carbone,
    - . un radical de formule :

$$-R_1-OH$$

$$-R_1-COOR_2$$

$$-R_1-CHO$$

$-R_1-NO_2$

$-R_1-CN$

$-R_1-N-(R_2)_2$

$-R_1-CO-N-(R_2)_2$

$-R_1-X$

$-R_1-CF_3$

dans lesdites formules, $R_1$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_2$ représente un atome d'hydrogène ou un radical alkyle linéaire ou ramifié ayant de 1 à 6 atomes de carbone ; X symbolise un atome d'halogène, de préférence un atome de chlore, de brome ou de fluor.

- les radicaux R' et R et les 2 atomes successifs du cycle benzénique peuvent former entre eux, un cycle ayant de 5 à 7 atomes, comprenant éventuellement un autre hétéroatome.

Lorsque n est supérieur ou égal à 1, les radicaux R' et R et les 2 atomes successifs du cycle benzénique peuvent être liés entre eux par un radical alkylène, alcénylène ou alcénylidène ayant de 2 à 4 atomes de carbone pour former un hétérocycle saturé, insaturé ou aromatique ayant de 5 à 7 atomes de carbone. Un ou plusieurs atomes de carbone peuvent être remplacés par un autre hétéroatome, de préférence l'oxygène. Ainsi, les radicaux OR' et R peuvent représenter un radical méthylène dioxy ou éthylène dioxy.
Dans la formule (Ia), R' représente préférentiellement un radical alkyle, linéaire ou ramifié, ayant de 1 à 4 atomes de carbone, de préférence, un radical méthyle ou éthyle.
L'éther aromatique de formule (I) peut porter un ou plusieurs substituants R.
R représente plus préférentiellement, l'un des atomes ou groupes suivants :

. un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
. un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy, isobutoxy, sec-butoxy, tert-butoxy,
. un atome d'halogène, de préférence un atome de fluor, chlore ou brome, un radical trifluorométhyle.

Dans la formule (Ia), R représente préférentiellement un radical alkoxy, linéaire ou ramifié, ayant de 1 à 4 atomes de carbone, de préférence, un radical méthoxy ou éthoxy.
Le procédé de l'invention s'applique plus particulièrement, aux éthers aromatiques substitués c'est-à-dire à des éthers aromatiques de formule (I) ou (Ia) dans lesquelles n est au moins égal à 1.
Interviennent préférentiellement dans le procédé de l'invention des éthers aromatiques de formule (I) ou (Ia) dans lesquelles :

- n est au moins égal à 1,
- R' représente un radical alkyle, linéaire ou ramifié, ayant de 1 à 6 atomes de carbone ou un radical phényle,
- R représente un radical alkoxy, linéaire ou ramifié, ayant de 1 à 4 atomes de carbone, de préférence, un radical méthoxy ou éthoxy.
- les radicaux OR' et R forment un radical méthylène dioxy ou éthylène dioxy.

Le procédé de l'invention s'applique plus particulièrement aux éthers aromatiques de formule (Ia) dans laquelle n est égal à 1, les radicaux R et OR' représentant tous deux, des radicaux alkoxy, identiques ou différents.

A titre illustratif de composés répondant à la formule (I), on peut mentionner plus particulièrement :

- les monoéthers non substitués tels que l'anisole, l'éthoxybenzène (phénétole), le propoxybenzène, l'isopropoxybenzène, le butoxybenzène, l'isobutoxybenzène, le 1-méthoxynaphtalène, le 2-méthoxynaphtalène, le 2-éthoxynaphtalène ; les monoéthers substitués tels que le 2-chloroanisole, le 3-chloroanisole, le 2-bromoanisole, le 3-bromoanisole, le 2-méthylanisole, le 3-méthylanisole, le 2-éthylanisole, le 3-éthylanisole, le 2-isopropylanisole, le 3-isopropylanisole, le 2-propylanisole, le 3-propylanisole, le 2-allylanisole, le 2-butylanisole, le 3-butylanisole, le 2-benzylanisole, le 2-cyclohexylanisole, le 1-bromo-2-éthoxybenzène, le 1-bromo-3-éthoxybenzène, le 1-chloro-2-éthoxybenzène, le 1-chloro-3-éthoxybenzène, le 1-éthoxy-2-éthylbenzène, le 1-éthoxy-3-éthylbenzène, le 1-méthoxy-2-allyloxybenzène, le 2,3-diméthylanisole, le 2,5-diméthylanisole,
- les diéthers comme le vératrole, le 1,3-diméthoxybenzène, le 1,4-diméthoxybenzène, le 1,2-diéthoxy- benzène, le 1,3-diéthoxybenzène, le 1,2-dipropoxybenzène, le 1,3-dipropoxy- benzène, le 1,2-méthylènedioxybenzène, le 1,2-éthylènedioxybenzène,
- les triéthers comme le 1,2,3-triméthoxybenzène, le 1,3,5-triméthoxybenzène, le 1,3,5-triéthoxybenzène.

Les composés auxquels s'applique de manière plus particulièrement intéressante le procédé selon l'invention, sont les éthers substitués répondant à la formule (I) ou (la) dans lesquelles n est au moins égal à 1. L'invention est bien adaptée pour effectuer l'acylation du vératrole.
Pour ce qui est du réactif d'acylation, il est choisi dans le groupe formé par les halogénures d'acides carboxyliques et les anhydrides d'acides carboxyliques.
Les acides carboxyliques sont des acides carboxyliques aliphatiques, saturés ou insaturés, linéaires ou ramifiés ou des acides cycloaliphatiques, éventuellement substitués, saturés ou insaturés.
Ils répondent plus particulièrement à la formule (II) :

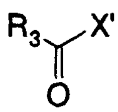
(II)

dans laquelle :

- $R_3$ représente :
  . un radical aliphatique saturé ou insaturé, linéaire ou ramifié ayant de 1 à 24 atomes de carbone ; un radical cycloaliphatique, saturé ou insaturé, monocyclique ou polycyclique, ayant de 3 à 12 atomes de carbone ;
- X' représente:
  . un atome d'halogène, de préférence un atome de chlore ou de brome,
  . un radical -O-CO-$R_4$ avec $R_4$, identique ou différent de $R_3$, ayant la même signification que $R_3$ : $R_3$ et $R_4$ pouvant former ensemble un radical divalent aliphatique saturé ou insaturé, linéaire ou ramifié, ayant au moins 2 atomes de carbone.

Plus préférentiellement, $R_3$ représente un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome (par exemple, l'oxygène), par un groupe fonctionnel (par exemple -CO-) et/ou porteuse de substituants (par exemple, des atomes d'halogène ou un groupe $CF_3$).
$R_3$ représente également un radical alcényle ayant de 2 à 10 atomes de carbone, tel que vinyle, propèn-yle, butèn-yle, pentèn-yle, hexèn-yle, octèn-yle, décèn-yle.
Le radical $R_3$ représente également un radical non aromatique, de préférence, cycloaliphatique, par exemple, un radical cyclohexyle, qui peut être éventuellement substitué. N'importe quel substituant peut être présent sur le cycle dans la mesure où il n'interfère pas au niveau du produit souhaité.
Comme exemples plus particuliers de substituants, on peut citer, notamment :

. un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
. un radical alkoxy linéaire ou ramifié ayant de 1 à 6 atomes de carbone, de préférence de 1 à 4 atomes de carbone

tel que les radicaux méthoxy, éthoxy, propoxy, isopropoxy, butoxy, isobutoxy, sec-butoxy, tert-butoxy,
. un groupe hydroxyle,
. un atome d'halogène, de préférence un atome de fluor, chlore ou brome.

Les agents d'acylation préférés sont les anhydrides d'acides. Ils répondent plus particulièrement à la formule (II) dans laquelle $R_3$ et $R_4$ sont identiques et représentent un radical alkyle ayant de 1 à 4 atomes de carbone, éventuellement porteurs d'atomes d'halogène, de préférence, de chlore.
Lorsque l'agent d'acylation est un halogénure d'acide, il répond préférentiellement à la formule (II) dans laquelle X' représente un atome de chlore et $R_3$ représente un radical alkyle ayant de 1 à 4 atomes de carbone, de préférence, méthyle ou éthyle éventuellement porteurs d'atomes d'halogène, de préférence, de chlore.
A titre illustratif d'agents d'acylation répondant à la formule (II), on peut citer plus particulièrement :

- l'anhydride acétique,
- l'anhydride propanoïque,
- l'anhydride isobutyrique,
- l'anhydride trifluoroacétique,
- l'anhydride de monochloracétyle,
- l'anhydride de dichloroacétyle,
- le chlorure d'acétyle,
- le chlorure de monochloracétyle,
- le chlorure de dichloroacétyle,
- le chlorure de propanoyle,
- le chlorure d'isobutanoyle,
- le chlorure de pivaloyle,
- le chlorure de crotonyle.

Conformément à l'invention, la réaction d'acylation est conduite avantageusement en phase liquide comprenant l'éther aromatique et l'agent d'acylation et en présence du catalyseur.
L'un des réactifs de départ peut servir de solvant réactionnel mais il est également possible de faire appel à un solvant organique.
Comme exemples de solvants convenant à la présente invention, on peut citer en particulier les hydrocarbures aliphatiques ou aromatiques, halogénés ou non, les éther-oxydes aliphatiques, cycloaliphatiques ou aromatiques ou des solvants aprotiques, plus polaires.
A titre d'exemples d'hydrocarbures aliphatiques ou cycloaliphatiques, on peut citer plus particulièrement les paraffines tels que notamment, l'hexane, l'heptane, l'octane, le nonane, le décane, le undécane, le dodécane, le tétradécane ou le cyclohexane, et les hydrocarbures aromatiques comme notamment le benzène, le toluène, les xylènes, le cumène, les coupes pétrolières constituées de mélange d'alkylbenzènes notamment les coupes de type Solvesso® .
En ce qui concerne les hydrocarbures halogénés aliphatiques ou aromatiques, on peut mentionner plus particulièrement, les hydrocarbures perchlorés tels que notamment le tétrachloroéthylène, l'hexachloroéthane ; les hydrocarbures partiellement chlorés tels que le dichlorométhane, le chloroforme, le 1,2-dichloroéthane, le 1,1,1-trichloroéthane, le 1,1,2,2-tétrachloroéthane, le pentachloroéthane, le trichloroéthylène, le 1-chlorobutane, le 1,2-dichlorobutane; le monochlorobenzène, le 1,2-dichlorobenzène, le 1,3-dichlorobenzène, le 1,4-dichlorobenzène, le 1,2,4-trichlorobenzène ou des mélanges de différents chlorobenzènes ; le bromoforme, le bromoéthane ou le 1,2-dibromoéthane ; le monobromobenzène ou des mélanges de monobromobenzène avec un ou plusieurs dibromobenzènes ; le 1-bromonaphtalène.
On peut utiliser également à titre de solvants organiques, les éther-oxydes aliphatiques, cycloaliphatiques ou aromatiques et, plus particulièrement, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou 1,2-diméthoxyéthane), le diméthyléther du diéthylèneglycol (ou 1,5-diméthoxy 3-oxapentane) ; l'oxyde de biphényle ou de benzyle ; le dioxane, le tétrahydrofuranne (THF).
Comme exemples de solvants organiques aprotiques, plus polaires qui peuvent également être mis en oeuvre dans le procédé de l'invention, on peut citer plus particulièrement les carboxamides linéaires ou cycliques comme le N,N-diméthylacétamide (DMAC), le N,N-diéthylacétamide, le diméthylformamide (DMF), le diéthylformamide ou la 1-méthyl-2-pyrrolidinone (NMP) ; le diméthylsulfoxyde (DMSO) ; les sulfones cycliques ou non telles que la tétraméthylsulfone, la diméthylsulfone ; l'hexaméthylphosphotriamide (HMPT) ; les urées tétrasubstituées cycliques ou non comme la diméthyléthylèneurée, la diméthylpropylèneurée, la tétraméthylurée.
Les solvants préférés sont : le dichlorométhane, le tétrachlorométhane, le THF et l'oxyde de diéthyle.
On peut également utiliser un mélange de solvants organiques.

On utilise préférentiellement, le substrat de départ comme solvant réactionnel.
Comme mentionné précédemment, l'éther aromatique est mis à réagir avec un agent d'acylation, éventuellement en présence d'un solvant réactionnel tel que défini et en présence d'un catalyseur solide défini ci-avant.
Le rapport entre le nombre de moles d'éther aromatique et le nombre de moles d'agent d'acylation peut varier car le substrat peut servir de solvant réactionnel. Ainsi, le rapport peut aller de 0,1 à 20, et se situe de préférence entre 0,5 et 10.
La quantité de catalyseur que l'on met en oeuvre dans le procédé de l'invention peut varier dans de larges limites.
Lorsque l'on réalise le procédé en discontinu, le catalyseur peut représenter en poids par rapport à l'éther aromatique engagé, de 0,01 à 50 %, de préférence, de 1,0 à 20 %. Cependant si l'on réalise le procédé en continu, par exemple en faisant réagir un mélange de l'éther aromatique et de l'agent d'acylation sur un lit fixe de catalyseur, ces rapports catalyseur/éther aromatique n'ont pas de sens et à un instant donné, on pourra avoir un excès pondéral de catalyseur par rapport à l'éther aromatique de départ.
Pour ce qui est de la quantité de solvant organique mis en oeuvre, elle est choisie généralement de telle sorte que le rapport entre le nombre de moles de solvant organique et le nombre de moles d'éther aromatique varie, de préférence, entre 0 et 100 et encore plus préférentiellement entre 0 et 50.
Il est également possible de conduire le procédé de l'invention, en présence d'eau : cette dernière pouvant représenter de 0 à 10 % du poids de l'agent d'acylation.
La température à laquelle est mise en oeuvre la réaction d'acylation dépend de la réactivité du substrat de départ et de celle de l'agent d'acylation.
Elle se situe entre 20°C et 300°C, de préférence entre 40°C et 200°C.
Généralement, la réaction est conduite à pression atmosphérique mais des pressions plus faibles ou plus élevées peuvent également convenir. On travaille sous pression autogène lorsque la température de réaction est supérieure à la température d'ébullition des réactifs et/ou des produits.
D'un point de vue pratique, le procédé peut être mis en oeuvre en discontinu ou en continu.
Selon la première variante, il n'y a pas de contraintes au niveau de la mise en oeuvre des réactifs. Ils peuvent être introduits dans un ordre quelconque.
Après mise en contact des réactifs, on porte le mélange réactionnel à la température souhaitée.
L'autre variante de l'invention consiste à conduire la réaction en continu, dans un réacteur tubulaire comportant le catalyseur solide disposé en lit fixe.
L'éther aromatique et l'agent d'acylation peuvent être introduits séparément ou en mélange dans le réacteur.
Ils peuvent également être introduits dans un solvant tel que mentionné précédemment.
Le temps de séjour du flux de matière sur le lit catalytique varie, par exemple, entre 15 mn et 10 heures, et de préférence, entre 30 mn et 5 heures.
En fin de réaction, on obtient une phase liquide comprenant l'éther aromatique acylé qui peut être récupéré de manière classique, par distillation ou par recristallisation dans un solvant approprié, après élimination préalable des réactifs en excès.
Le procédé de l'invention est particulièrement bien adapté à la préparation de la 3,4-diméthoxyacétophénone dénommée couramment acétovératrole, par acétylation du vératrole.
Un avantage du procédé de l'invention est que la réaction d'acylation s'effectue sans qu'il y ait une O-désalkylation de l'éther aromatique de départ.
Les exemples qui suivent, illustrent l'invention sans toutefois la limiter.
Les exemples 2 à 4, 9 et 10 sont donnés à titre comparatif.
Dans les exemples, les rendements mentionnés correspondent à la définition suivante :

$$\text{Rendement : } RR_{A.A.} = \frac{\text{nombre de moles d'agent d'acylation introduites}}{\text{nombre de moles de composé aromatique acylé formées}} \%$$

<u>Exemple 1 :</u>

Dans cet exemple, on prépare un catalyseur selon l'invention dont la phase active est une zéolithe Y, de type structural FAU, ayant un paramètre de maille *a* = 24,29 Angström et dont les caractéristiques sont les suivantes :

- le rapport Si/Al global est égal à 15,2,
- le rapport Si/Al de charpente égal à 20,
- le rapport atomique $Na/Al^{IV}$ = 0,018.

Le rapport $Na/Al^{IV}$ est déterminé par rapport à l'aluminium ($Al^{IV}$) présent dans la charpente de la zéolithe et

en faisant l'hypothèse que tout le sodium se trouve en position cationique.

Acétylation du vératrole

Dans un réacteur fermé de 30 ml, on charge 5,5 g de vératrole (40 mmol), 1,1 g d'anhydride acétique et 0,11 g de zéolithe préalablement calcinée à 550°C sous flux d'air sec.
Le réacteur est alors chauffé à 90°C.
Après 6 heures, le mélange réactionnel est filtré puis analysé par chromatographie en phase gazeuse.
Les résultats catalytiques sont rassemblés dans le tableau (I).

Exemple 2*:

Dans cet exemple, donné à titre comparatif, la phase active est une zéolithe ZSM-5, de type structural MFI, dont le rapport Si/Al global est égal à 27.

Acétylation du vératrole

L'acétylation du vératrole est réalisée dans les conditions opératoires décrites dans l'exemple 1. Les résultats catalytiques sont rassemblés dans le tableau (I).

Exemple 3* :

Dans cet exemple comparatif, la phase active est une zéolithe mordénite, de type structural MOR, dont le rapport Si/Al global est égal à 100.

Acétylation du vératrole

L'acétylation du vératrole est réalisée dans les conditions opératoires décrites dans l'exemple 1. Les résultats catalytiques sont rassemblés dans le tableau (I).

Exemple 4* :

Dans cet exemple comparatif, la phase active est une zéolithe Béta, de type structural BEA, dont le rapport Si/Al global est égal à 12,5.

Acétylation du vératrole

L'acétylation du vératrole est réalisée dans les conditions opératoires décrites dans l'exemple 1. Les résultats catalytiques sont rassemblés dans le tableau (I).

Tableau (I)

| N°exemple | Catalyseur | Rapport Si/Al global | $RR_{A.A.}$ (%) |
|---|---|---|---|
| 1 | H-Y | 15,2 | 95 |
| 2* | HZSM5 | 27 | 12 |
| 3* | H-mordénite | 100 | 25 |
| 4* | Hβ | 12,5 | 53 |

Le tableau 1 met en évidence l'intérêt qu'il y a à utiliser un catalyseur selon l'invention, à savoir une zéolithe Y qui permet d'obtenir un rendement élevé en acétovératrole.

Exemple 5 :

Dans cet exemple, on prépare un catalyseur conformément à l'invention.
On place en suspension 20 g de zéolithe Y sous forme Na, de rapport Si/Al global égal à 2,8, dans 100 ml d'une solution aqueuse de nitrate d'ammonium 10 N ($NH_4NO_3$).

On chauffe la suspension à reflux durant 3 heures.

On sépare la zéolithe par filtration puis on la lave à l'eau distillée. Ce cycle d'opérations est opéré successivement 4 fois.

On obtient une zéolithe $NH_4$-Y qui est soumise à une calcination à 725°C en présence de vapeur d'eau et en lit traversé durant 4 heures.

On la met ensuite en suspension dans 100 ml d'une solution d'acide nitrique 2 N et l'on porte le mélange à reflux durant 2 heures.

On sépare la zéolithe par filtration puis on la lave à l'eau distillée jusqu'à obtention d'eaux de lavage dont le pH est équivalent à celui de l'eau distillée.

Les caractéristiques de la zéolithe H-Y désaluminée qui est ainsi obtenue, sont reportées dans le tableau (II).

Acétylation du vératrole

Dans un réacteur fermé de 30 ml, on charge 5,5 g de vératrole (40 mmol), 1,1 g d'anhydride acétique et 0,11 g de la zéolithe préalablement calcinée à 550°C sous flux d'air sec.

Le réacteur est alors chauffé à 90°C.

Après 6 heures, le mélange réactionnel est filtré puis analysé par chromatrographie en phase gazeuse.

Les résultats catalytiques sont rassemblés dans le tableau (III).

Exemple 6 :

Dans cet exemple, on prépare un catalyseur conformément à l'invention.

On place en suspension 20 g de zéolithe Y sous forme Na, de rapport Si/Al global égal à 2,8, dans 100 ml d'une solution aqueuse de nitrate d'ammonium, 10 N ($NH_4NO_3$).

On chauffe la suspension à reflux durant 4 heures.

On sépare la zéolithe par filtration puis on la lave à l'eau distillée

La zéolithe Y possède alors une teneur en sodium de 2,4 % poids.

On obtient une zéolithe Y partiellement échangée qui est soumise à une calcination à 725°C en présence de vapeur d'eau et en lit traversé durant 4 heures.

On la met ensuite en suspension dans 100 ml d'une solution d'acide nitrique 1,2 N et l'on porte le mélange à reflux durant 2 heures.

On sépare la zéolithe par filtration puis on la lave à l'eau distillée jusqu'à obtention d'eaux de lavage dont le pH est équivalent à celui de l'eau distillée.

Puis, on la soumet à trois échanges ioniques par des solutions de nitrate d'ammonium 10 N en utilisant le même mode opératoire que celui décrit au début de la préparation.

Les caractéristiques de la zéolithe H-Y désaluminée qui est ainsi obtenue, sont reportées dans le tableau (II).

Acétylation du vératrole

L'acétylation du vératrole est réalisée dans les conditions opératoires décrites dans l'exemple 5. Les résultats sont rassemblés dans le tableau (III).

Exemple 7 :

Dans cet exemple, on prépare un catalyseur conformément à l'invention.

On place en suspension 20 g de zéolithe Y sous forme Na, de rapport Si/Al global égal à 2,7 dans 100 ml d'une solution aqueuse de nitrate d'ammonium, 10 N ($NH_4NO_3$).

On chauffe la suspension à reflux durant 3 heures.

On sépare la zéolithe par filtration puis on la lave à l'eau distillée. Ce cycle d'opérations est opéré successivement 4 fois.

On obtient une zéolithe $NH_4$-Y qui est soumise à une calcination à 750°C en présence de vapeur d'eau et en lit traversé durant 4 heures.

On la met ensuite en suspension dans 100 ml d'une solution d'acide nitrique 2,5 N et l'on porte le mélange à reflux durant 3 heures.

On sépare la zéolithe par filtration puis on la lave à l'eau distillée jusqu'à obtention d'eaux de lavage dont le pH est équivalent à celui de l'eau distillée.

Les caractéristiques de la zéolithe H-Y désaluminée qui est ainsi obtenue, sont reportées dans le tableau (II).

Acétylation du vératrole

L'acétylation du vératrole est réalisée dans les conditions opératoires décrites dans l'exemple 5. Les résultats sont rassemblés dans le tableau (III).

Exemple 8:

Dans cet exemple, on prépare un catalyseur conformément à l'invention.

On place en suspension 20 g de zéolithe Y sous forme Na, de rapport Si/Al global égal à 2,7 dans 100 ml d'une solution aqueuse de nitrate d'ammonium 10 N ($NH_4NO_3$).

On chauffe la suspension à reflux durant 3 heures.

On sépare la zéolithe par filtration puis on la lave à l'eau distillée. Ce cycle d'opérations est opéré successivement 4 fois.

On obtient une zéolithe $NH_4$-Y qui est soumise à une calcination à 770°C en présence de vapeur d'eau et en lit traversé durant 4 heures.

On la met ensuite en suspension dans 100 ml d'une solution d'acide nitrique 1,5 N et l'on porte le mélange à reflux durant 3 heures.

On sépare la zéolithe par filtration puis on la lave à l'eau distillée jusqu'à obtention d'eaux de lavage dont le pH est équivalent à celui de l'eau distillée.

On réalise une attaque acide avec une solution d'acide nitrique 2N réalisée dans les mêmes conditions opératoires que celles décrites précédemment.

Les caractéristiques de la zéolithe H-Y désaluminée qui est ainsi obtenue, sont reportées dans le tableau (II).

Acétylation du vératrole

L'acétylation du vératrole est réalisée dans les conditions opératoires décrites dans l'exemple 5. Les résultats sont rassemblés dans le tableau (III).

Exemple 9* :

Dans cet exemple donné à titre comparatif, la zéolithe Y utilisée est fournie par PQ Zéolites sous la référence CBV50001.

Les caractéristiques de cette zéolithe H-Y, déterminées par les mêmes méthodes analytiques que celles utilisées pour caractériser les zéolithes des exemples 1 à 8, sont reportées dans le tableau (II).

Acétylation du vératrole

L'acétylation du vératrole est réalisée dans les conditions opératoires décrites dans l'exemple 5. Les résultats sont rassemblés dans le tableau (III).

Exemple 10* :

Dans cet exemple donné à titre comparatif, la zéolithe Y utilisée est fournie par Degussa sous la référence TC134.

Les caractéristiques de cette zéolithe H-Y, déterminées par les mêmes méthodes analytiques que celles utilisées pour caractériser les zéolithes des exemples 1 à 9, sont reportées dans le tableau (II).

Tableau (II)

| N°exemple | Paramètre de la maille *a* (Angström) | Si/Al atomique global (FX) | Si/Al$^{(IV)}$ de charpente | Na/Al$^{(IV)}$(3) (% atomique) |
|---|---|---|---|---|
| 5 | 24,29 | 13,5 | 29(1) | 5,7 |

(1) Le rapport Si/Al$^{(IV)}$ de charpente est déterminé par diffraction des rayons X. Cette technique permet la mesure du paramètre de maille de la zéolithe Y, puis l'utilisation de la relation de Fichter-Schmittler (Fichter-Schmittler, H., Loshe, U., Engelhardt, G. et Patzelova, V. Cryst. Res. Tech. 1984, 19, K1) permet d'accéder au rapport Si/Al$^{(IV)}$ de charpente.

(3) Le rapport Na/Al$^{(IV)}$ est déterminé par rapport à l'aluminium [Al$^{(IV)}$] présent dans la charpente et en formant l'hypothèse que tout le sodium se trouve en position cationique.

Tableau (II) (suite)

| N°exemple | Paramètre de la maille *a* (Angström) | Si/Al atomique global (FX) | Si/Al$^{(IV)}$ de charpente | Na/Al$^{(IV)}$(3) (% atomique) |
|---|---|---|---|---|
| 6 | 24,35 | 5,5 | 13,6(1) | 2,3 |
| 7 | 24,26 | 17,8 | 27(2) | 1,9 |
| 8 | 24,26 | 33,8 | 45(1) | 2,2 |
| 9* | 24,53 | 3,3 | 4,8(1) | 1,4 |
| 10* | 24,29 | 11,6 | 23(2) | 26,2 |

(1) Le rapport Si/Al$^{(IV)}$ de charpente est déterminé par diffraction des rayons X. Cette technique permet la mesure du paramètre de maille de la zéolithe Y, puis l'utilisation de la relation de Fichter-Schmittler (Fichter-Schmittler, H., Loshe, U., Engelhardt, G. et Patzelova, V. Cryst. Res. Tech. 1984, 19, K1) permet d'accéder au rapport Si/Al$^{(IV)}$ de charpente.

(2) Le rapport Si/Al$^{(IV)}$ de charpente est déterminé par RMN du solide du $^{29}Si$ et par spectroscopie infrarouge.

(3) Le rapport Na/Al$^{(IV)}$ est déterminé par rapport à l'aluminium [Al$^{(IV)}$] présent dans la charpente et en formant l'hypothèse que tout le sodium se trouve en position cationique.

Acétylation du vératrole

Dans un réacteur fermé de 30 ml, on charge 5,5 g de vératrole (40 mmol), 1.1 g d'anhydride acétique (11 mmol) et 0,11 g de catalyseur décrit dans les exemples 5 à 10.

Le réacteur est alors chauffé à 90°C.

Après 6 heures, le mélange réactionnel est filtré puis analysé par chromatrographie en phase gazeuse.

Les résultats catalytiques sont rassemblés dans le tableau (III).

Tableau (III)

| Référence du catalyseur | $RR_{A.A.}$ (%) |
|---|---|
| exemple 5 | 89 |
| exemple 6 | 95 |
| exemple 7 | 97 |
| exemple 8 | 91 |
| exemple 9* | 22 |
| exemple 10* | 36 |

L'examen de ce tableau met en évidence le gain apporté par la mise en oeuvre des zéolithes Y ayant les caractéristiques conformes à la présente invention.

Exemple 11 :

Dans cet exemple, on met en oeuvre la zéolithe Y décrite dans l'exemple 5.

Acétylation de l'anisole

Dans un réacteur fermé de 30 ml, on charge 5,81 g d'anisole (53 mmol), 1,1 g d'anhydride acétique (10,7 mmol) et 0,11 g de catalyseur.

Le réacteur est alors chauffé à 90°C.

Après 6 heures, le mélange réactionnel est filtré puis analysé par chromatrographie en phase gazeuse.

Le rendement obtenu en 4-méthoxyacétophénone (ou acétoanisole) est :

$RR_{AA}$ = 69 %.

## Revendications

**1.** Procédé d'acylation d'un éther aromatique qui consiste à faire réagir ledit éther avec un agent d'acylation, en

présence d'un catalyseur zéolithique, ledit procédé étant caractérisé par le fait que l'on conduit la réaction d'acylation en présence d'une quantité efficace d'un catalyseur comprenant une zéolithe de type faujasite ou zéolithe Y ayant les caractéristiques physico-chimiques suivantes :

- un rapport atomique dénommé "$Si/Me^1$ global", entre le nombre d'atomes de l'élément silicium et le nombre d'atomes de tout élément trivalent $Me^1$ contenu dans la zéolithe, compris entre 2,4 et 90, de préférence entre 2,4 et 75 et de manière encore plus préférée, entre 2,4 et 60,
- une teneur en métal alcalin $Me^2$ telle que le rapport atomique $Me^2/Me^{1(IV)}$, entre le nombre d'atomes de métal alcalin $Me^2$ et le nombre d'atomes de tout élément trivalent $Me^{1(IV)}$ inclus dans le réseau zéolithique, est inférieur à 0,2, de préférence inférieur à 0,1 et de manière encore plus préférée, inférieur à 0,05.

**2.** Procédé selon la revendication 1 caractérisé par le fait que la zéolithe de type faujasite ou zéolithe Y présente un rapport atomique dénommé "$Si/Me^{1(IV)}$ de charpente zéolithique", entre le nombre d'atomes de l'élément silicium et le nombre d'atomes de tout élément trivalent $Me^{1(IV)}$ inclus dans le réseau zéolithique compris entre 5 et 100, de préférence entre 6 et 80 et de manière encore plus préférée, entre 8 et 60.

**3.** Procédé selon les revendications 1 et 2 caractérisé par le fait que l'éther aromatique répond à la formule générale (I):

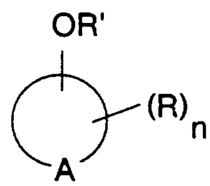

(I)

dans laquelle :

- A symbolise le reste d'un cycle formant tout ou partie d'un système carbocyclique aromatique, monocyclique ou polycyclique, système comprenant au moins un groupe OR' : ledit reste cyclique pouvant porter un ou plusieurs substituants,
- R représente un ou plusieurs substituants, identiques ou différents,
- R' représente un radical hydrocarboné ayant de 1 à 24 atomes de carbone, qui peut être un radical aliphatique acyclique saturé ou insaturé, linéaire ou ramifié ; un radical cycloaliphatique saturé, insaturé ou aromatique, monocyclique ou polycyclique ; un radical aliphatique saturé ou insaturé, linéaire ou ramifié, porteur d'un substituant cyclique,
- R' et R peuvent former un cycle comprenant éventuellement un autre hétéroatome,
- n est un nombre inférieur ou égal à 4.

**4.** Procédé selon l'une des revendications 1 à 3 caractérisé par le fait que l'éther aromatique répond à la formule générale (I) dans laquelle R' représente :

- un radical aliphatique acyclique, saturé ou insaturé, linéaire ou ramifié, de préférence un radical alkyle linéaire ou ramifié ayant de 1 à 12 atomes de carbone, de préférence de 1 à 6 atomes de carbone : la chaîne hydrocarbonée pouvant être éventuellement interrompue par un hétéroatome, un groupe fonctionnel et/ou porteuse d'un substituant,
- un radical aliphatique acyclique, saturé ou insaturé, linéaire ou ramifié porteur d'un substituant cyclique éventuellement substitué : ledit radical acyclique pouvant être relié au cycle par un lien valentiel, un hétéroatome ou un groupe fonctionnel,
- un radical carbocyclique saturé ou comprenant 1 ou 2 insaturations dans le cycle, ayant généralement de 3 à 8 atomes de carbone, de préférence, 6 atomes de carbone dans le cycle ; ledit cycle pouvant être substitué.
- un radical carbocyclique aromatique, de préférence monocyclique ayant généralement au moins 4 atomes de carbone, de préférence, 6 atomes de carbone dans le cycle ; ledit cycle pouvant être substitué.

**5.** Procédé selon la revendication 1 caractérisé par le fait que l'éther aromatique répond à la formule générale (I) dans laquelle R' représente un radical alkyle linéaire ou ramifié ayant de 1 à 4 atomes de carbone, de préférence un radical méthyle, éthyle ou un radical phényle.

**6.** Procédé selon l'une des revendications 1 à 5 caractérisé par le fait que l'éther aromatique répond à la formule générale (I) dans laquelle le reste A représente le reste d'un composé carbocyclique aromatique, monocyclique ayant au moins 4 atomes de carbone et de préférence 6 atomes de carbone ou le reste d'un composé carbocyclique polycyclique, de préférence un reste naphtalénique : le reste A pouvant porter un ou plusieurs substituants sur le noyau aromatique.

**7.** Procédé selon l'une des revendications 1 à 6 caractérisé par le fait que l'éther aromatique répond à la formule (Ia) :

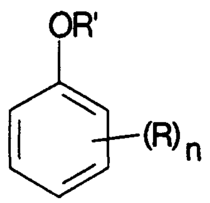

(Ia)

dans laquelle :

- n est un nombre inférieur ou égal à 4, de préférence égal à 1 ou 2,
- le radical R' représente un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle ou un radical phényle,
- le ou les radicaux R représentent l'un des atomes ou groupes suivants :

  . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  . un radical cyclohexyle ou benzyle,
  . un groupe acyle ayant de 2 à 6 atomes de carbone,
  . un radical de formule :

$$-R_1-OH$$

$$-R_1-COOR_2$$

$$-R_1-CHO$$

$$-R_1-NO_2$$

$$-R_1-CN$$

$$-R_1-N-(R_2)_2$$

$$-R_1-CO-N-(R_2)_2$$

$-R_1-X$

$-R_1-CF_3$

dans lesdites formules, $R_1$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_2$ représente un atome d'hydrogène ou un radical alkyle linéaire ou ramifié ayant de 1 à 6 atomes de carbone ; X symbolise un atome d'halogène, de préférence un atome de chlore, de brome ou de fluor.

- les radicaux R' et R et les 2 atomes successifs du cycle benzénique peuvent former entre eux, un cycle ayant de 5 à 7 atomes, comprenant éventuellement un autre hétéroatome.

**8.** Procédé selon la revendication 7 caractérisé par le fait que l'éther aromatique répond à la formule (Ia) dans laquelle n est supérieur ou égal à 1, les radicaux R' et R et les 2 atomes successifs du cycle benzénique peuvent être liés entre eux par un radical alkylène, alcénylène ou alcénylidène ayant de 2 à 4 atomes de carbone pour former un hétérocycle saturé, insaturé ou aromatique ayant de 5 à 7 atomes de carbone dans lequel un ou plusieurs atomes de carbone peuvent être remplacés par un hétéroatome, de préférence l'oxygène : les radicaux OR' et R formant de préférence un radical méthylène dioxy ou éthylène dioxy.

**9.** Procédé selon les revendications 1 et 2 caractérisé par le fait que l'éther aromatique est un éther aromatique substitué répondant à la formule (I) ou (Ia) dans lesquelles :

- n est au moins égal à 1,
- R' représente un radical alkyle, linéaire ou ramifié, ayant de 1 à 6 atomes de carbone ou un radical phényle,
- R représente un radical alkoxy, linéaire ou ramifié, ayant de 1 à 4 atomes de carbone, de préférence, un radical méthoxy ou éthoxy.
- les radicaux OR' et R forment un radical méthylène dioxy ou éthylène dioxy.

**10.** Procédé selon la revendication 7 caractérisé par le fait que l'éther aromatique répond à la formule (Ia) dans laquelle n est égal à 1, les radicaux R et OR' représentant tous deux, des radicaux alkoxy, identiques ou différents, ledit éther étant de préférence, le vératrole.

**11.** Procédé selon l'une des revendications 1 à 10 caractérisé par le fait que l'agent d'acylation répond à la formule (II) :

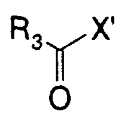

(II)

dans laquelle :

- $R_3$ représente :
  . un radical aliphatique saturé ou insaturé, linéaire ou ramifié ayant de 1 à 24 atomes de carbone ; un radical cycloaliphatique, saturé ou insaturé, monocyclique ou polycyclique, ayant de 3 à 12 atomes de carbone ;
- X' représente :
  . un atome d'halogène, de préférence un atome de chlore ou de brome,
  . un radical $-O-CO-R_4$ avec $R_4$, identique ou différent de $R_3$, ayant la même signification que $R_3$ : $R_3$ et $R_4$ pouvant former ensemble un radical divalent aliphatique saturé ou insaturé, linéaire ou ramifié, ayant au moins 2 atomes de carbone.

**12.** Procédé selon la revendication 11 caractérisé par le fait que l'agent d'acylation répond à la formule (II) dans laquelle

X' représente un atome de chlore et $R_3$ représente un radical alkyle linéaire ou ramifié ayant de 1 à 12 atomes de carbone, de préférence, 1 à 6 atomes : la chaîne hydrocarbonée pouvant être éventuellement interrompue par un hétéroatome ou par un groupe fonctionnel ou porteuse de substituants, de préférence, d'atomes d'halogène ; X' représente un radical -O-CO-$R_4$. dans laquelle $R_3$ et $R_4$ sont identiques et représentent un radical alkyle ayant de 1 à 4 atomes de carbone éventuellement porteur d'atomes d'halogène.

**13.** Procédé selon la revendication 11 et 12 caractérisé par le fait que l'agent d'acylation est choisi parmi :

- l'anhydride acétique,
- l'anhydride propanoïque,
- l'anhydride isobutyrique,
- l'anhydride trifluoroacétique,
- l'anhydride de monochloracétyle,
- l'anhydride de dichloroacétyle,
- le chlorure d'acétyle,
- le chlorure de monochloracétyle,
- le chlorure de dichloroacétyle,
- le chlorure de propanoyle,
- le chlorure d'isobutanoyle,
- le chlorure de pivaloyle,
- le chlorure de crotonyle.

**14.** Procédé selon l'une des revendications 1 à 13 caractérisé par le fait que le catalyseur est une zéolithe Y ou zéolithe de type faujasite ayant, à l'état déshydraté, la composition chimique correspondant à la formule empirique suivante :

$$(1\text{-}x)\ H_2O,\ x[(Me^2)_2O],\ [(Me^1)_2O_3],\ 2n\ SiO_2 \qquad \text{(F1)}$$

dans ladite formule:

- $Me^1$ représente tout élément ayant un degré d'oxydation de +3, de préférence, l'aluminium, la gallium, le fer, le bore et leurs mélanges,
- $Me^2$ représente un métal choisi dans le groupe des éléments de la colonne 1a et leurs mélanges, de préférence les métaux alcalins tels que le lithium, le sodium, le potassium, le rubidium et le césium,
- n, égal au rapport atomique "Si/$Me^1$ global" défini précédemment, est compris entre 2,4 et 90, de préférence entre 2,4 et 75 et de manière encore plus préférée, entre 2,4 et 60,
- x, égal au rapport atomique $Me^2/Me^{1(IV)}$ défini précédemment, est inférieur à 0,2, de préférence inférieur à 0,1 et de manière encore plus préférée, compris entre 0 et 0,05.

**15.** Procédé selon la revendication 14 caractérisé par le fait que le catalyseur est une zéolithe répondant à la formule chimique (F1) dans laquelle $Me^1$ représente l'aluminium et $Me^2$ le sodium et/ou le potassium.

**16.** Procédé selon l'une des revendications 14 et 15 caractérisé par le fait que la zéolithe mise en oeuvre a un paramètre de maille *a* inférieur à 24,50 Angström, de préférence, compris entre 24,23 et 24,42 Angstrôm.

**17.** Procédé selon l'une des revendications 1 à 16 caractérisé par le fait que la zéolithe Y est soumise à un traitement de désalumination de telle sorte que les rapports atomiques Si/$Me^1$ définis précédemment soient compris dans les intervalles précités.

**18.** Procédé selon la revendication 17 caractérisé par le fait que les traitements de désalumination sont les calcinations en présence de vapeur, les calcinations en présence de vapeur d'eau suivies d'attaques par des acides minéraux ($HNO_3$, HCl...), les traitements de désalumination par des réactifs tels que le tétrachlorure de silicium ($SiCl_4$), l'hexafluorosilicate d'ammonium ($(NH_4)_2SiF_6$) ou bien encore l'éthylènediaminetétracétique (EDTA) ainsi que sa forme mono ou disodique ; l'attaque acide directe par des solutions d'acides minéraux ou d'acides organiques ou toute combinaison desdites méthodes.

**19.** Procédé selon l'une des revendications 1 à 18 caractérisé par le fait que la zéolithe est mise en oeuvre seule ou en mélange avec une matrice minérale choisie, de préférence, parmi les oxydes de métaux, tels que les oxydes d'aluminium, de silicium et/ou de zirconium, ou encore parmi les argiles et plus particulièrement, le kaolin, le talc ou la montmorillonite.

**20.** Procédé selon l'une des revendications 1 à 19 caractérisé par le fait que l'on met en oeuvre un solvant organique choisi parmi :

- les hydrocarbures aliphatiques, cycloaliphatiques ou aromatiques.
- les hydrocarbures halogénés aliphatiques ou aromatiques,
- les éther-oxydes aliphatiques, cycloaliphatiques ou aromatiques,
- les carboxamides linéaires ou cycliques,
- le diméthylsulfoxyde (DMSO),
- les sulfones cycliques ou non,
- l'hexaméthylphosphotriamide (HMPT),
- les urées tétrasubstituées cycliques ou non.

**21.** Procédé selon l'une des revendications 1 à 20 caractérisé par le fait que le rapport entre le nombre de moles d'éther aromatique et le nombre de moles d'agent d'acylation varie entre 0,1 et 20, et se situe de préférence entre 0,5 et 10.

**22.** Procédé selon l'une des revendications 1 à 21 caractérisé par le fait que la quantité de catalyseur représente en poids par rapport à l'éther aromatique engagé, de 0,01 à 50 %, de préférence, de 1,0 à 20 %.

**23.** Procédé selon l'une des revendications 1 à 22 caractérisé par le fait que la température à laquelle est mise en oeuvre la réaction d'acylation se situe entre 20°C et 300°C, de préférence entre 40°C et 200°C.

**24.** Zéolithe Y ou zéolithe de type faujasite, ayant à l'état déshydraté, la composition chimique correspondant à la formule empirique suivante :

$$(1\text{-}x)H_2O,\ x[(Me^2)_2O],\ [(Me^1)_2O_3],\ 2n\ SiO_2 \qquad (F1)$$

dans ladite formule :

- $Me^1$ représente tout élément ayant un degré d'oxydation de +3, de préférence, l'aluminium, la gallium, le fer, le bore et leurs mélanges,
- $Me^2$ représente un métal choisi dans le groupe des éléments de la colonne 1a et leurs mélanges, de préférence les métaux alcalins tels que le lithium, le sodium, le potassium, le rubidium et le césium,
- n, égal au rapport atomique "Si/$Me^1$ global" défini précédemment, est compris entre 2,4 et 90, de préférence entre 2,4 et 75 et de manière encore plus préférée, entre 2,4 et 60,
- x, égal au rapport atomique $Me^2/Me^{1(IV)}$ défini précédemment, est inférieur à 0,2, de préférence inférieur à 0,1 et de manière encore plus préférée, compris entre 0 et 0,05.

et caractérisée par le fait qu'elle a un paramètre de maille *a* inférieur à 24,50 Angström, de préférence, compris entre 24,23 et 24,42 Angström.

**25.** Zéolithe selon la revendication 24 caractérisée par le fait qu'elle répond à la formule chimique (F1) dans laquelle $Me^1$ représente l'aluminium et $Me^2$ représente le sodium et/ou le potassium.

**26.** Catalyseur d'acylation d'un éther aromatique comprenant la zéolithe décrite dans l'une des revendications 24 et 25.

**Patentansprüche**

**1.** Verfahren zur Acylierung eines aromatischen Ethers, das darin besteht, den genannten Ether mit einem Acylierungsmittel in Gegenwart eines zeolithischen Katalysators reagieren zu lassen, wobei das genannte Verfahren

dadurch gekennzeichnet ist, daß man die Acylierungsreaktion in Gegenwart einer wirksamen Menge eines Katalysators durchführt, der einen Zeolith vom Faujasit-Typ oder einen Zeolith Y mit den folgenden physikalisch-chemischen Eigenschaften enthält:

- ein als "Si/$Me^1$ global" bezeichnetes Atomverhältnis zwischen der Zahl der Atome des Elements Silicium und der Zahl der Atome aller im Zeolith enthaltenen dreiwertigen Elemente $Me^1$, das zwischen 2,4 und 90, vorzugsweise zwischen 2,4 und 75 und noch spezieller zwischen 2,4 und 60, liegt,
- einen Gehalt an Alkalimetall $Me^2$, so daß das Atomverhältnis $Me^2/Me^{1(IV)}$ zwischen der Zahl der Atome des Alkalimetalls $Me^2$ und der Zahl der Atome aller im Zeolithgitter enthaltenen dreiwertigen Elemente $Me^{1(IV)}$ kleiner als 0,2, vorzugsweise kleiner als 0,1 und noch spezieller kleiner als 0,05, ist.

**2.** Verfahren nach Anspruch 1, dadurch gekennzeichnet, daß der Zeolith vom Faujasit-Typ oder der Zeolith Y ein mit "Si/$Me^{1(IV)}$ des Zeolithgerüsts" bezeichnetes Atomverhältnis zwischen der Zahl der Atome des Elements Silicium und der Zahl der Atome aller im Zeolithgitter enthaltenen dreiwertigen Elemente $Me^{1(IV)}$ zwischen 5 und 100, vorzugsweise zwischen 6 und 80 und noch spezieller zwischen 8 und 60, aufweist.

**3.** Verfahren nach Anspruch 1 oder 2, dadurch gekennzeichnet, daß der aromatische Ether der allgemeinen Formel (I) entspricht:

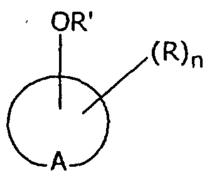

(I)

in der:

- A den Rest eines Rings bedeutet, der ganz oder teilweise ein aromatisches, monocyclisches oder polycyclisches carbocyclisches System bildet, wobei das System mindestens eine OR'-Gruppe enthält: wobei der genannte cyclische Rest einen oder mehrere Substituenten tragen kann,
- R einen oder mehrere gleiche oder verschiedene Substituenten bedeutet,
- R' einen Kohlenwasserstoffrest mit 1 bis 24 Kohlenstoffatomen bedeutet, der ein acyclischer gesättigter oder ungesättigter, linearer oder verzweigter aliphatischer Rest sein kann; ein gesättigter, ungesättigter oder aromatischer, monocyclischer oder polycyclischer cycloaliphatischer Rest; ein gesättigter oder ungesättigter, linearer oder verzweigter aliphatischer Rest, der Träger eines cyclischen Substituenten ist,
- R' und R einen Ring mit gegebenenfalls einem weiteren Heteroatom bilden können,
- n eine Zahl kleiner oder gleich 4 ist.

**4.** Verfahren nach einem der Ansprüche 1 bis 3, dadurch gekennzeichnet, daß der aromatische Ether der allgemeinen Formel (I) entspricht, in der R' bedeutet:

- einen acyclischen, gesättigten oder ungesättigten, linearen oder verzweigten aliphatischen Rest, vorzugsweise einen linearen oder verzweigten Alkylrest mit 1 bis 12 Kohlenstoffatomen, vorzugsweise mit 1 bis 6 Kohlenstoffatomen: wobei die Kohlenwasserstoffkette gegebenenfalls durch ein Heteroatom, eine funktionelle und/oder eine einen Substituenten tragende Gruppe unterbrochen sein kann,
- einen acyclischen, gesättigten oder ungesättigten, linearen oder verzweigten aliphatischen Rest, der Träger eines cyclischen, gegebenenfalls substituierten Substituenten ist: wobei der genannte acyclische Rest durch ein valentes Bindeglied, ein Heteroatom oder eine funktionelle Gruppe mit dem Ring verbunden sein kann,
- einen gesättigten oder 1 oder 2 ungesättigte Stellen im Ring aufweisenden carbocyclischen Rest mit im allgemeinen 3 bis 8 Kohlenstoffatomen, vorzugsweise 6 Kohlenstoffatomen, im Ring; wobei der genannte Ring substituiert sein kann,
- einen aromatischen, vorzugsweise monocyclischen, carbocyclischen Rest mit im allgemeinen mindestens 4 Kohlenstoffatomen, vorzugsweise 6 Kohlenstoffatomen, im Ring; wobei der genannte Ring substituiert sein kann.

**5.** Verfahren nach Anspruch 1, dadurch gekennzeichnet, daß der aromatische Ether der allgemeinen Formel (I) entspricht, in der R' einen linearen oder verzweigten Alkylrest mit 1 bis 4 Kohlenstoffatomen, vorzugsweise einen Methyl-, Ethylrest oder einen Phenylrest, bedeutet.

**6.** Verfahren nach einem der Ansprüche 1 bis 5, dadurch gekennzeichnet, daß der aromatische Ether der allgemeinen Formel (I) entspricht, in der der Rest A den Rest einer aromatischen, monocyclischen carbocyclischen Verbindung mit mindestens 4 Kohlenstoffatomen und vorzugsweise 6 Kohlenstoffatomen oder den Rest einer polycyclischen carbocyclischen Verbindung, vorzugsweise einen Naphthalinrest, bedeutet: wobei der Rest A einen oder mehrere Substituenten auf dem aromatischen Ring tragen kann.

**7.** Verfahren nach einem der Ansprüche 1 bis 6, dadurch gekennzeichnet, daß der aromatische Ether der Formel (Ia) entspricht:

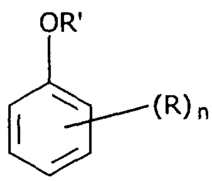

(Ia)

in der:

- n eine Zahl kleiner oder gleich 4, vorzugsweise gleich 1 oder 2, ist,
- der Rest R' einen linearen oder verzweigten Alkylrest mit 1 bis 6 Kohlenstoffatomen, vorzugsweise 1 bis 4 Kohlenstoffatomen, wie einen Methyl-, Ethyl-, Propyl-, Isopropyl-, Butyl-, Isobutyl-, sec.-Butyl-, tert.-Butylrest oder einen Phenylrest bedeutet,
- der oder die Reste R eines der folgenden Atome oder Gruppen bedeuten:
  - einen linearen oder verzweigten Alkylrest mit 1 bis 6 Kohlenstoffatomen, vorzugsweise mit 1 bis 4 Kohlenstoffatomen, wie einen Methyl-, Ethyl-, Propyl-, Isopropyl-, Butyl-, Isobutyl-, sec.-Butyl-, tert.-Butylrest,
  - einen linearen oder verzweigten Alkenylrest mit 2 bis 6 Kohlenstoffatomen, vorzugsweise mit 2 bis 4 Kohlenstoffatomen, wie einen Vinyl-, Allylrest,
  - einen linearen oder verzweigten Alkoxyrest mit 1 bis 6 Kohlenstoffatomen, vorzugsweise mit 1 bis 4 Kohlenstoffatomen, wie die Dimethoxy-, Ethoxy-, Propoxy-, Isopropoxy-, Butoxyreste,
  - einen Cyclohexyl- oder Benzylrest,
  - eine Acylgruppe mit 2 bis 6 Kohlenstoffatomen,
  - einen Rest der Formel:

$$-R_1-OH$$

$$-R_1-COOR_2$$

$$-R_1-CHO$$

$$-R_1-NO_2$$

$$-R_1-CN$$

$$-R_1-N-(R_2)_2$$

$$-R_1-CO-N-(R_2)_2$$

$$-R_1-X$$

$$-R_1-CF_3$$

wobei in den genannten Formeln $R_1$ ein valentes Bindeglied oder einen divalenten, linearen oder verzweigten, gesättigten oder ungesättigten Kohlenwasserstoffrest mit 1 bis 6 Kohlenstoffatomen bedeutet, wie z.B. einen Methylen-, Ethylen-, Propylen-, Isopropylen-, Isopropylidenrest; $R_2$ ein Wasserstoffatom oder einen linearen oder verzweigten Alkylrest mit 1 bis 6 Kohlenstoffatomen bedeutet; X ein Halogenatom, vorzugsweise ein Chlor-, Brom- oder Fluoratom, bedeutet;

- die Reste R' und R und die 2 aufeinanderfolgenden Atome des Benzolrings miteinander einen Ring mit 5 bis 7 Atomen bilden können, der gegebenenfalls ein weiteres Heteroatom aufweist.

**8.** Verfahren nach Anspruch 7, dadurch gekennzeichnet, daß der aromatische Ether der Formel (Ia) entspricht, in der n größer oder gleich 1 ist, die Reste R' und R und die 2 aufeinanderfolgenden Atome des Benzolrings untereinander durch einen Alkylen-, Alkenylen- oder Alkenylidenrest mit 2 bis 4 Kohlenstoffatomen verbunden sein können, um einen gesättigten, ungesättigten oder aromatischen Heteroring mit 5 bis 7 Kohlenstoffatomen zu bilden, in dem ein oder mehrere Kohlenstoffatome durch ein Heteroatom, vorzugsweise Sauerstoff, ersetzt werden können: wobei die Reste OR' und R vorzugsweise einen Methylendioxy- oder Ethylendioxyrest bilden.

**9.** Verfahren nach Anspruch 1 oder 2, dadurch gekennzeichnet, daß der aromatische Ether ein substituierter aromatischer Ether ist, der der Formel (I) oder (Ia) entspricht, in denen:

- n mindestens gleich 1 ist,
- R' einen linearen oder verzweigten Alkylrest mit 1 bis 6 Kohlenstoffatomen oder einen Phenylrest bedeutet,
- R einen linearen oder verzweigten Alkoxyrest mit 1 bis 4 Kohlenstoffatomen, vorzugsweise einen Methoxy- oder Ethoxyrest, bedeutet,
- die Reste OR' und R einen Methylendioxy- oder Ethylendioxyrest bedeuten.

**10.** Verfahren nach Anspruch 7, dadurch gekennzeichnet, daß der aromatische Ether der Formel (Ia) entspricht, in der n gleich 1 ist, die Reste R und OR' beide gleiche oder verschiedene Alkoxyreste bedeuten, wobei der genannte Ether vorzugsweise Veratrol ist.

**11.** Verfahren nach einem der Ansprüche 1 bis 10, dadurch gekennzeichnet, daß das Acylierungsmittel der Formel (II) entspricht:

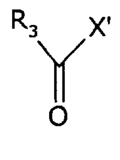

**(II)**

in der:

- $R_3$ bedeutet:
    - einen gesättigten oder ungesättigten, linearen oder verzweigten aliphatischen Rest mit 1 bis 24 Kohlenstoffatomen; einen gesättigten oder ungesättigten, monocyclischen oder polycyclischen cycloaliphatischen Rest mit 3 bis 12 Kohlenstoffatomen;
- X' bedeutet:

- ein Halogenatom, vorzugsweise ein Chlor- oder Bromatom,
- einen -O-CO-$R_4$-Rest mit $R_4$, das gleich mit oder unterschiedlich zu $R_3$ ist und dieselbe Bedeutung wie $R_3$ hat: wobei $R_3$ und $R_4$ zusammen einen gesättigten oder ungesättigten, linearen oder verzweigten divalenten aliphatischen Rest mit mindestens 2 Kohlenstoffatomen bilden können.

**12.** Verfahren nach Anspruch 11, dadurch gekennzeichnet, daß das Acylierungsmittel der Formel (II) entspricht, in der X' ein Chloratom und $R_3$ einen linearen oder verzweigten Alkylrest mit 1 bis 12 Kohlenstoffatomen, vorzugsweise 1 bis 6 Atomen, bedeutet: wobei die Kohlenwasserstoffkette gegebenenfalls durch ein Heteroatom oder durch eine funktionelle oder Substituenten, vorzugsweise Halogenatome, tragende Gruppe unterbrochen sein kann; X' einen -O-CO-$R_4$.-Rest bedeutet, in der $R_3$ und $R_4$ gleich sind und einen Alkylrest mit 1 bis 4 Kohlenstoffatomen bedeuten, der gegebenenfalls Träger von Halogenatomen ist.

**13.** Verfahren nach Anspruch 11 oder 12, dadurch gekennzeichnet, daß das Acylierungsmittel gewählt ist aus:

- Essigsäureanhydrid
- Propionsäureanhydrid
- Isobuttersäureanhydrid
- Trifluoressigsäureanhydrid
- Monochloracetylanhydrid
- Dichloracetylanhydrid
- Acetylchlorid
- Monochloracetylchlorid
- Dichloracetylchorid
- Propanoylchlorid
- Isobuttersäurechlorid
- Pivaloylchlorid
- Crotonoylchlorid

**14.** Verfahren nach einem der Ansprüche 1 bis 13, dadurch gekennzeichnet, daß der Katalysator einen Zeolith Y oder einen Zeolith vom Faujasit-Typ ist, der im dehydrisierten Zustand die chemische Zusammensetzung hat, die der folgenden empirischen Formel entspricht:

$$(1-x)\ H_2O,\ x\ [(Me^2)_2O],\ [(Me^1)_2O_3],\ 2n\ SiO_2 \qquad (F1)$$

wobei in der genannten Formel:

- $Me^1$ alle Elemente mit einem Oxidationsgrad von +3, vorzugsweise Aluminium, Gallium, Eisen, Bor und deren Gemische, bedeutet,
- $Me^2$ ein Metall bedeutet, das aus der Gruppe der Elemente der Spalte 1a und deren Gemischen, vorzugsweise den Alkalimetallen wie Lithium, Natrium, Kalium, Rubidium und Cäsium, gewählt ist,
- n gleich dem zuvor definierten Atomverhältnis "Si/$Me^1$ global" ist und zwischen 2,4 und 90, vorzugsweise zwischen 2,4 und 75 und noch spezieller zwischen 2,4 und 60, liegt,
- x gleich dem zuvor definierten Atomverhältnis $Me^2/Me^{1(IV)}$ ist und kleiner als 0,2, vorzugsweise kleiner als 0,1, ist und noch spezieller zwischen 0 und 0,05 liegt.

**15.** Verfahren nach Anspruch 14, dadurch gekennzeichnet, daß der Katalysator ein Zeolith ist, der der chemischen Formel (F1) entspricht, in der $Me^1$ Aluminium und $Me^2$ Natrium und/oder Kalium bedeuten.

**16.** Verfahren nach Anspruch 14 oder 15, dadurch gekennzeichnet, daß der verwendete Zeolith eine Maschenweite *a* von weniger als 24,50 Angström, vorzugsweise zwischen 24,23 und 24,42 Angström, aufweist.

**17.** Verfahren nach einem der Ansprüche 1 bis 16, dadurch gekennzeichnet, daß der Zeolith Y einer Dealuminierungsbehandlung unterzogen wird, so daß die zuvor beschriebenen Atomverhältnisse Si/$Me^1$ in den zuvor genannten Intervallen liegen.

**18.** Verfahren nach Anspruch 17, dadurch gekennzeichnet, daß die Dealuminierungsbehandlungen Calcinierungen

in Gegenwart von Dampf, Calcinierungen in Gegenwart von Wasserdampf gefolgt von Angriffen durch mineralische Säuren ($HNO_3$, HCl...), Dealuminierungsbehandlungen durch Reagenzien wie Siliciumtetrachlorid ($SiCl_4$), Ammoniumhexafluorsilicat ($(NH_4)_2SiF_6$) oder auch Ethylendiamintetraessigsäure (EDTA) sowie seine Mono- oder Dinatriumform sind; wobei es sich um einen direkten Säureangriff durch Lösungen mineralischer Säuren oder organischer Säuren oder jede Kombination der genannten Methoden handelt.

**19.** Verfahren nach einem der Ansprüche 1 bis 18, dadurch gekennzeichnet, daß der Zeolith allein oder als Gemisch mit einer mineralischen Matrix verwendet wird, die vorzugsweise aus den Metalloxiden wie den Aluminium-, Silicium-und/oder Zirkoniumoxiden oder auch aus den Tonen und noch spezieller Kaolin, Talk oder Montmorillonit gewählt ist.

**20.** Verfahren nach einem der Ansprüche 1 bis 19, dadurch gekennzeichnet, daß man ein organisches Lösungsmittel verwendet, das gewählt ist aus:

- den aliphatischen, cycloaliphatischen oder aromatischen Kohlenwasserstoffen,
- den aliphatischen oder aromatischen halogenierten Kohlenwasserstoffen,
- den aliphatischen, cycloaliphatischen oder aromatischen Etheroxiden,
- den linearen oder cyclischen Carboxyamiden,
- Dimethylsulfoxid (DMSO)
- den cyclischen oder nicht cyclischen Sulfonen,
- Hexamethylphosphotriamid (HMPT),
- den cyclischen oder nicht cyclischen tetrasubstituierten Harnstoffen.

**21.** Verfahren nach einem der Ansprüche 1 bis 20, dadurch gekennzeichnet, daß das Verhältnis zwischen der Molzahl des aromatischen Ethers und der Molzahl des Acylierungsmittels zwischen 0,1 und 20 schwankt und vorzugsweise zwischen 0,5 und 10 liegt.

**22.** Verfahren nach einem der Ansprüche 1 bis 21, dadurch gekennzeichnet, daß die Menge des Katalysators gewichtsbezogen im Verhältnis zum beteiligten aromatischen Ether 0,01 bis 50 %, vorzugsweise 1,0 bis 20 %, beträgt.

**23.** Verfahren nach einem der Ansprüche 1 bis 22, dadurch gekennzeichnet, daß die Temperatur, bei der die Acylierungsreaktion durchgeführt wird, zwischen 20 °C und 300 °C, vorzugsweise zwischen 40 °C und 200 °C, liegt.

**24.** Zeolith Y oder Zeolith vom Faujasit-Typ, der im dehydrisierten Zustand die chemische Zusammensetzung hat, die der folgenden empirischen Formel entspricht:

$$(1\text{-}x)\ H_2O,\ x[(Me^2)_2O],\ [(Me^1)_2O_3],\ 2n\ SiO_2 \qquad (F1)$$

wobei in der genannten Formel:

- $Me^1$ alle Elemente mit einem Oxidationsgrad von +3, vorzugsweise Aluminium, Gallium, Eisen, Bor und deren Gemische, bedeutet,
- $Me^2$ ein Metall bedeutet, das aus der Gruppe der Elemente der Spalte 1a und deren Gemischen, vorzugsweise den Alkalimetallen wie Lithium, Natrium, Kalium, Rubidium und Cäsium, gewählt ist,
- n gleich dem zuvor definierten Atomverhältnis "Si/$Me^1$ global" ist und zwischen 2,4 und 90, vorzugsweise zwischen 2,4 und 75 und noch spezieller zwischen 2,4 und 60, liegt,
- x gleich dem zuvor definierten Atomverhältnis $Me^2/Me^{1(IV)}$ ist und kleiner als 0,2, vorzugsweise kleiner als 0,1, ist und noch spezieller zwischen 0 und 0,05 liegt,

und dadurch gekennzeichnet, daß er eine Maschenweite *a* von weniger als 24,50 Angström, vorzugsweise zwischen 24,23 und 24,42 Angström, aufweist.

**25.** Zeolith nach Anspruch 24, dadurch gekennzeichnet, daß er der chemischen Formel (F1) entspricht, in der $Me^1$ Aluminium und $Me^2$ Natrium und/oder Kalium bedeuten.

**26.** Katalysator zur Acylierung eines aromatischen Ethers, der einen in den Ansprüchen 24 oder 25 beschriebenen

Zeolith enthält.

## Claims

**1.** A process for the acylation of an aromatic ether which consists in reacting said ether with an acylation agent in the presence of a zeolitic catalyst, said process being characterised in that the acylation reaction is carried out in the presence of an effective quantity of a catalyst comprising a faujasite type zeolite or a Y zeolite with the following physico-chemical characteristics:

- a so-called "global Si/$Me^1$" atomic ratio between the number of atoms of the element silicon and the number of atoms of every trivalent element $Me^1$ contained in the zeolite, within the range of 2.4 to 90, preferably within the range of 2.4 to 75, and still more preferably within the range of 2.4 to 60,
- an $Me^2$ alkali metal content such that the $Me^2/Me^{1(IV)}$ atomic ratio between the number of atoms of alkali metal $Me^2$ and the number of atoms of every trivalent element $Me^{1(IV)}$ included in the zeolitic network is less than 0.2, preferably less than 0.1, and still more preferably less than 0.05.

**2.** A process according to claim 1, characterised in that the faujasite type zeolite or Y zeolite has a so-called "Si/$Me^{1(IV)}$ atomic ratio of the zeolite framework" between the number of atoms of the element silicon and the number of atoms of every trivalent element $Me^{1(IV)}$ included in the zeolitic network within the range of 5 to 100, preferably within the range of 6 to 80, and still more preferably within the range of 8 to 60.

**3.** A process according to claims 1 and 2, characterised in that the aromatic ether corresponds to general formula (I):

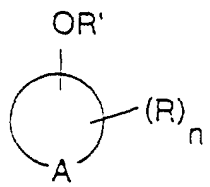

(I)

wherein:

- A represents the residue of a cycle forming all or a portion of a monocyclic or polycyclic aromatic carbocyclic system, which system contains at least one $OR^1$ group : said cyclic residue may carry one or more substituents,
- R represents one or more substituents which may be identical or different;
- R' represents a hydrocarbon radical having from 1 to 24 carbon atoms, which may be a saturated or unsaturated, linear or branched acyclic aliphatic radical; a monocyclic or polycyclic, saturated, unsaturated or aromatic cycloaliphatic radical; a saturated or unsaturated linear or branched aliphatic radical carrying a cyclic substituent;
- R' and R may form a cycle which may contain a further heteroatom;
- n is a number less than or equal to 4.

**4.** A process according to one of claims 1 to 3, characterised in that the aromatic ether corresponds to the general formula (I) where R' represents:

- a saturated or unsaturated, linear or branched acyclic aliphatic radical, preferably a linear or branched alkyl radical having 1 to 12 carbon atoms, preferably 1 to 6 carbon atoms: the hydrocarbon chain may be interrupted by a heteroatom, a functional group and/or may carry a substituent;
- a saturated or unsaturated, linear or branched acyclic aliphatic radical carrying a cyclic substituent which may be substituted: said acyclic radical may be bonded to the cycle by a valence bond, a heteroatom, or a functional group;
- a carbocyclic radical which may be saturated or contain 1 or 2 unsaturations in the cycle, generally having 3 to 8 carbon atoms, preferably 6 carbon atoms in the cycle; said cycle may be substituted.
- an aromatic carbocyclic radical, preferably a monocyclic radical generally having at least 4 carbon atoms, preferably 6 carbon atoms in the cycle; said cycle may be able to be substituted.

**5.** A process according to claim 1, characterised in that the aromatic ether corresponds to general formula (I) in which R' represents a linear or branched alkyl radical having 1 to 4 carbon atoms, preferably a methyl, ethyl or phenyl radical.

**6.** A process according to one of claims 1 to 5, characterised in that the aromatic ether corresponds to general formula (I) where the residue A represents the residue of a monocyclic aromatic carbocyclic compound having at least 4 carbon atoms and preferably 6 carbon atoms or the residue of a polycyclic carbocyclic compound, preferably a naphthalenic residue: residue A may carry one or more substituents on the aromatic nucleus.

**7.** A process according to one of claims 1 to 6, characterised in that the aromatic ether corresponds to formula (Ia):

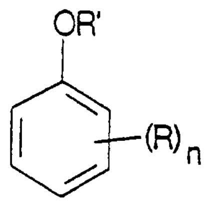

(Ia)

where:

- n is a number less than or equal to 4, preferably equal to 1 or 2;
- the radical R' represents a linear or branched alkyl radical having 1 to 6 carbon atoms, preferably 1 to 4 carbon atoms, such as methyl, ethyl, propyl, isopropyl, butyl, isobutyl, sec-butyl, tert-butyl, or a phenyl radical;
- the R radical(s) represents/represent one of the following atoms or groups:
  - a linear or branched alkyl radical having 1 to 6 carbon atoms, preferably 1 to 4 carbon atoms, such as methyl, ethyl, propyl, isopropyl, butyl, isobutyl, sec-butyl, tert-butyl;
  - a linear or branched alkenyl radical having 2 to 6 carbon atoms, preferably 2 to 4 carbon atoms, such as vinyl, allyl;
  - a linear or branched alkoxy radical having 1 to 6 carbon atoms, preferably 1 to 4 carbon atoms, such as methoxy, ethoxy, propoxy, isopropoxy, butoxy radicals;
  - a cyclohexyl or benzyl radical;
  - an acyl group having 2 to 6 carbon atoms;
  - a radical of the formula:

$$-R_1-OH$$

$$-R_1-COOR_2$$

$$-R_1-CHO$$

$$-R_1-NO_2$$

$$-R_1-CN$$

$$-R_1-N-(R_2)_2$$

$$-R_1-CO-N-(R_2)_2$$

$-R_1-X$

$-R_1-CF_3$

in which formulae, $R_1$ represents a valence bond or a saturated or unsaturated, linear or branched divalent hydrocarbon radical having 1 to 6 carbon atoms, such as methylene, ethylene, propylene, isopropylene, or isopropylidene; $R_2$ represents a hydrogen atom or a linear or branched alkyl radical having 1 to 6 carbon atoms; and X symbolises a halogen atom, preferably a chlorine, bromine or fluorine atom;

. radicals R and R and the 2 successive atoms of the benzene ring can form between them a cycle having 5 to 7 carbon atoms which may contain a further heteroatom.

**8.** A process according to claim 7, characterised in that the aromatic ether corresponds to formula (Ia) where n is greater than or equal to 1, radicals R and R' and the 2 successive atoms of the benzene ring can be bonded together by an alkylene, alkenylene or alkenylidene radical having 2 to 4 carbon atoms to form a saturated, unsaturated or aromatic heterocycle having 5 to 7 carbon atoms in which one or more carbon atoms can be replaced by a heteroatom, preferably oxygen: the radicals OR' and R preferably forming a dioxymethylene or dioxyethylene radical.

**9.** A process according to claims 1 and 2, characterised in that the aromatic ether is a substituted aromatic ether corresponding to formula (I) or (Ia), where:

- n is at least equal to 1;
- R' represents a linear or branched alkyl radical having 1 to 6 carbon atoms or a phenyl radical;
- R represents a linear or branched alkoxy radical having 1 to 4 carbon atoms, preferably a methoxy or ethoxy radical.
- radicals OR' and R form a dioxymethylene or dioxyethylene radical.

**10.** A process according to claim 7, characterised in that the aromatic ether corresponds to formula (Ia) where n equals 1, radicals R and OR' both represent alkoxy radicals which may be identical or different, said ether preferably being veratrol

**11.** A process according to one of claims 1 to 10, characterised in that the acylation agent corresponds to formula (II):

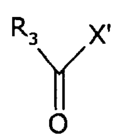

(II)

where:

- $R^3$ represents
    - a saturated or unsaturated, linear or branched aliphatic radical having 1 to 24 carbon atoms; a saturated or unsaturated, monocyclic or polycyclic cycloaliphatic radical having 3 to 12 carbon atoms;
- X' represents
    - a halogen atom, preferably a chlorine or bromine atom;
    - a -O-CO-$R_4$ radical where $R_4$, which may be identical or different to $R_3$, has the same meaning as $R_3$; $R_3$ and $R_4$ may together form a saturated or unsaturated, linear or branched aliphatic divalent radical having at least 2 carbon atoms.

**12.** A process according to claim 11, characterised in that the acylation agent corresponds to formula (II) where X'

represents a chlorine atom and $R_3$ represents a linear or branched alkyl radical having 1 to 12 carbon atoms, preferably 1 to 6 atoms: the hydrocarbon chain may be interrupted by a heteroatom or by a functional group or may carry substituents, preferably halogen atoms; X' represents a -O-CO-$R_4$ radical where $R_3$ and $R_4$ are identical and represent an alkyl radical having 1 to 4 carbon atoms which may carry halogen atoms.

**13.** A process according to claims 11 and 12, characterised in that the acylation agent is selected from:

- acetic anhydride;
- propanoic anhydride;
- isobutyric anhydride;
- trifluoroacetic anhydride;
- monochloroacetyl anhydride;
- dichloroacetyl anhydride;
- acetyl chloride;
- monochloracetyl chloride;
- dichloroacetyl chloride;
- propanoyl chloride;
- isobutanoyl chloride;
- pivaloyl chloride;
- crotonyl chloride.

**14.** A process according to one of claims 1 to 13, characterised in that the catalyst is a Y zeolite or a faujasite type zeolite having, in its dehydrated state, a chemical composition corresponding to the following empirical formula:

$$(1\text{-}x)\ H_2O,\ x\ [(Me^2)_2O],\ [(Me^1)_2O_3],\ 2n\ SiO_2 \qquad (F1)$$

in which formula:

- $Me^1$ represents any element with a degree of oxidation of +3, preferably aluminium, gallium, iron, boron, and mixtures thereof;
- $Me^2$ represents a metal selected from the group of elements from column Ia and mixtures thereof, preferably alkali metals such as lithium, sodium, potassium, rubidium and caesium;
- n, equal to the "global Si/$Me^1$" atomic ratio defined hereinabove, is within the range of 2.4 to 90, preferably within the range of 2.4 to 75, and, still more preferably, within the range of 2.4 to 60,
- x, equal to the $Me^2/Me^{1(IV)}$ atomic ratio defined hereinabove, is less than 0.2, preferably less than 0.1, and, still more preferably within the range of 0 to 0.05.

**15.** A process according to claim 14, characterised in that the catalyst is a zeolite corresponding to chemical formula (F1) in which $Me^1$ represents aluminium and $Me^2$ represents sodium and/or potassium.

**16.** A process according to either claim 14 or claim 15, characterised in that the zeolite used has a lattice parameter *a* of less than 24.50 Angstrom, preferably within the range of 24.23 to 24.42 Angstrom.

**17.** A process according to any one of claims 1 to 16, characterised in that the Y zeolite undergoes a dealuminising treatment so that the Si/$Me^1$ atomic ratios defined hereinabove are within the afore-defined ranges.

**18.** A process according to claim 17, characterised in that the dealuminising treatments are calcination operations in the presence of steam, calcination operations in the presence of steam followed by attack with mineral acids ($HNO_3$, HCl...), dealuminising treatments using reactants such as silicon tetrachloride ($SiCl_4$), ammonium hexafluorosilicate ($(NH_4)_2SiF_6$), or ethylenediaminetetracetic acid (EDTA), or its mono or disodium form; direct acid attack using solutions of mineral acids or organic acids, or any combination of said methods.

**19.** A process according to one of claims 1 to 18, characterised in that the zeolite is used alone or mixed with a mineral matrix which is preferably selected from metal oxides, such as aluminium-, silicon- and/or zirconium oxides, or from clays, and more particularly kaolin, talc or montmorillonite.

**20.** A process according to one of claims 1 to 19, characterised in that an organic solvent is used which is selected from:

- aliphatic, cycloaliphatic or aromatic hydrocarbons;
- aliphatic or aromatic halogenated hydrocarbons;
- aliphatic, cycloaliphatic or aromatic ether-oxides;
- linear or cyclic carboxamides;
- dimethylsulphoxide (DMSO);
- cyclic or non cyclic sulphones;
- hexamethylphosphotriamide (HMPT);
- cyclic or non cyclic tetrasubstituted ureas.

**21.** A process according to one of claims 1 to 20, characterised in that the ratio between the number of moles of aromatic ether and the number of moles of acylation agent is between 0.1 and 20, and preferably between 0.5 and 10.

**22.** A process according to one of claims 1 to 21, characterised in that the quantity of catalyst represents 0.01% to 50% by weight, preferably 1.0% to 20%, in relation to the aromatic ether employed.

**23.** A process according to one of claims 1 to 22, characterised in that the temperature at which the acylation reaction is carried out is between 20°C and 300°C, preferably between 40°C and 200°C.

**24.** A Y zeolite or a faujasite type zeolite having in the dehydrated state a chemical composition corresponding to the following empirical formula:

$$(1\text{-}x)\ H_2O,\ x\ [(Me^2)_2O],\ [(Me^1)_2O_3],\ 2n\ SiO_2 \qquad (F1)$$

in which formula:

- $Me^1$ represents any element with a degree of oxidation of +3, preferably aluminium, gallium, iron, boron, and mixtures thereof,
- $Me^2$ represents a metal selected from the group of elements from column Ia and mixtures thereof, preferably alkali metals such as lithium, sodium, potassium, rubidium and caesium,
- n, equal to the "global Si/$Me^1$" atomic ratio defined hereinabove, is within the range of 2.4 to 90, preferably of 2.4 to 75, and still more preferably of 2.4 to 60,
- x, equal to the $Me^2/Me^{1(IV)}$ atomic ratio defined hereinabove, is less than 0.2, preferably less than 0.1, and still more preferably between 0 and 0.05,

and characterised in that it has a lattice parameter *a* of less than 24.50 Angstström, preferably within the range of 24.23 to 24.42 Angstström.

**25.** A zeolite according to claim 24, characterised in that it corresponds to the chemical formula (F1) in which $Me^1$ represents aluminium and $Me^2$ represents sodium and/or potassium.

**26.** An acylation catalyst for an aromatic ether, comprising the zeolite described in either claim 24 or claim 25.